# EUROPEAN PATENT SPECIFICATION

(11) **EP 3 744 325 B1**
(45) Date of publication and mention of the grant of the patent: **07.06.2023**
(21) Application number: 19740851.1
(22) Date of filing: 16.01.2019
(51) Int. Cl.: A61K 31/4709, A61P 35/00, A61K 33/243, A61P 35/04

(54) **APPLICATION OF NOVEL TYROSINE KINASE INHIBITOR, ANLOTINIB, IN OSTEOSARCOMA AND CHONDROSARCOMA**
VERWENDUNG DES NEUARTIGEN TYROSINKINASEHEMMERS ANLOTINIB IN OSTEOSARKOM UND CHONDROSARKOM
APPLICATION D'UN NOUVEL INHIBITEUR DE LA TYROSINE KINASE, ANLOTINIB, DANS L'OSTÉOSARCOME ET LE CHONDROSARCOME

(30) Priority: 22.01.2018 CN 201810058487
(43) Date of publication of application: 02.12.2020
(73) Proprietor: Chia Tai Tianqing Pharmaceutical Group Co., Ltd., Lianyungang, Jiangsu 222062 (CN)
(72) Inventor: HUA, Yingqi, Shanghai 200080 (CN); WANG, Gangyang, Shanghai 200080 (CN); CAI, Zhengdong, Shanghai 200080 (CN); WANG, Zhuoying, Shanghai 200080 (CN); ZHANG, Tao, Shanghai 200080 (CN); WANG, Hongsheng, Shanghai 200080 (CN); SUN, Mengxiong, Shanghai 200080 (CN); WANG, Xunqiang, Lianyungang, Jiangsu 222062 (CN); ZHAN, Xiaole, Lianyungang, Jiangsu 222062 (CN); YANG, Zhaoqiang, Lianyungang, Jiangsu 222062 (CN); TU, Lifan, Lianyungang, Jiangsu 222062 (CN); LI, Pu, Lianyungang, Jiangsu 222062 (CN)
(74) Representative: Grünecker Patent- und Rechtsanwälte PartG mbB
(86) International application number: PCT/CN2019/071846
(87) International publication number: WO 2019/141170

(56) References cited:
- CN-A- 107 970 241
- CN-A- 107 970 241
- YIHEBALI CHI ET AL: "Safety and Efficacy of Anlotinib, a Multikinase Angiogenesis Inhibitor, in Patients with Refractory Metastatic Soft-Tissue Sarcoma", CLINICAL CANCER RESEARCH, vol. 24, no. 21, 12 June 2018 (2018-06-12) , pages 5233-5238, XP055727667, US ISSN: 1078-0432, DOI: 10.1158/1078-0432.CCR-17-3766
- GUOSHUANG SHEN ET AL: "Anlotinib: a novel multi-targeting tyrosine kinase inhibitor in clinical development", JOURNAL OF HEMATOLOGY & ONCOLOGY, vol. 11, no. 1, 19 September 2018 (2018-09-19), pages 1-11, XP055700540, DOI: 10.1186/s13045-018-0664-7
- LUETKE ANJA ET AL: "Osteosarcoma treatment - Where do we stand? A state of the art re", CANCER TREATMENT REVIEWS, ELSEVIER, AMSTERDAM, NL, vol. 40, no. 4, 27 November 2013 (2013-11-27), pages 523-532, XP028625210, ISSN: 0305-7372, DOI: 10.1016/J.CTRV.2013.11.006
- ISAKOFF MICHAEL S. ET AL: "Osteosarcoma: Current Treatment and a Collaborative Pathway to Success", JOURNAL OF CLINICAL ONCOLOGY, vol. 33, no. 27, 20 September 2015 (2015-09-20), pages 3029-3035, XP055836398, ISSN: 0732-183X, DOI: 10.1200/JCO.2014.59.4895 Retrieved from the Internet: URL:https://www.ncbi.nlm.nih.gov/pmc/artic les/PMC4979196/pdf/zlj3029.pdf>
- BISHOP MICHAEL W. ET AL: "Future directions in the treatment of osteosarcoma", CURRENT OPINION IN PEDIATRICS., vol. 28, no. 1, 1 February 2016 (2016-02-01), pages 26-33, XP055836397, US ISSN: 1040-8703, DOI: 10.1097/MOP.0000000000000298 Retrieved from the Internet: URL:https://www.ncbi.nlm.nih.gov/pmc/artic les/PMC4761449/pdf/nihms-752279.pdf>
- BOVEE J V ET AL: "Emerging pathways in the development of chondrosarcoma of bone and implications for targeted treatment", THE LANCET ONCOLOGY, ELSEVIER, AMSTERDAM, NL, vol. 6, no. 8, 1 August 2005 (2005-08-01), pages 599-607, XP027624778, ISSN: 1470-2045 [retrieved on 2005-08-01]
- MACDONALD IONA J ET AL: "An update on current and future treatment options for chondrosarcoma", EXPERT REVIEW OF ANTICANCER THERAPY, vol. 19, no. 9, 2 September 2019 (2019-09-02), pages 773-786, XP055836386, GB ISSN: 1473-7140, DOI: 10.1080/14737140.2019.1659731 Retrieved from the Internet: URL:http://dx.doi.org/10.1080/14737140.201 9.1659731>
- TIAN ZHICHAO ET AL: "Retrospective review of the activity and safety of apatinib and anlotinib in patients with advanced osteosarcoma and soft tissue sarcoma", INVESTIGATION NEW DRUGS, MARTINUS NIJHOFF PUBLISHERS, BOSTON, US, vol. 38, no. 5, 25 February 2020 (2020-02-25), pages 1559-1569, XP037247578, ISSN: 0167-6997, DOI: 10.1007/S10637-020-00912-7 [retrieved on 2020-02-25]
- Tian Xin ; Lu Peng ; Wang Xiangjian: "Conference Report: Study on Anlotinib Hydrochloride and Its Reproductive Toxicity", 2017 Academic Exchange Conference on Reproductive Toxicology and Pharmacology Theory and Technology and Scientific and Technological Product Development, 31 August 2017 (2017-08-31), page 25, XP009522415, CN
- Chen, Xiaoguo et al.: "Research Progress of Targeted Therapy for Osteosarcoma", Journal of Military Surgeon in Southwest China, vol. 19, no. 5, 30 September 2017 (2017-09-30), pages 438-443, XP009522407, ISSN: 1672-7193, DOI: 10.3969/j.issn.1672-7193.2017.05.014

## Description

### TECHNICAL FIELD

The present disclosure relates to the technical field of drugs, specifically, a use of anlotinib in osteosarcoma and chondrosarcoma.

### BACKGROUND

Anlotinib is a small-molecule multi-target tyrosine kinase inhibitor which was developed by China and has independent intellectual property rights, and the publication number of the patent of the compound thereof is WO2008/112407. Clinical approval was obtained in 2011, the phase I clinical trial was completed in 2013, and clinical trials of anlotinib in a variety of cancers are currently underway, including non-small cell lung cancer, soft tissue sarcoma, gastric cancer, colorectal cancer, medullary thyroid carcinoma, differentiated thyroid carcinoma, esophageal squamous cell carcinoma, and the like.

The patent document (International Publication No.: WO2008/112407 and Chinese Patent No.: ZL200880007358.X) discloses the inhibitory activity of anlotinib on colon cancer, non-small cell lung cancer, liver cancer and breast cancer. However, the use of anlotinib in osteosarcoma has not been reported currently.

LUETKE ANJA et al (CANCER TREATMENT REVIEWS, vol. 40, no. 4, pages 523-532); ISAKOFF MICHAEL S. et al (JOURNAL OF CLINICAL ONCOLOGY, vol. 33, pages 3029-3035) and BISHOP MICHAEL W. et al (CURRENT OPINION IN PEDIATRICS., vol. 28, no. 1, pages 26-33) disclose and review the treatment options for osteosarcoma which do not include the compound anlotinib. BOVEE J V et al (THE LANCET ONCOLOGY, ELSEVIER, AMSTERDAM, NL, vol. 6, no. 8, pages 599-607) reviews the few available treatment options for chondrosarcoma (basically surgery) and points to possible promising targeted treatments.

### SUMMARY

The subject matter of the invention is as set out in the appended claims.

One aspect of the invention relates to Anlotinib or a pharmaceutically acceptable salt thereof, for use in inhibiting osteosarcoma and/or chondrosarcoma in a patient in need of treatment.

A further aspect of the invention relates to Anlotinib or a pharmaceutically acceptable salt thereof, for use as a potentiator of cisplatin in the inhibition of osteosarcoma and/or chondrosarcoma in a patient in need of treatment.

The purpose of the present disclosure is to provide a new use of anlotinib aiming at the defects in the prior art.

In order to achieve the above purpose, the technical solution adopted by the present disclosure is the use of anlotinib or a pharmaceutically acceptable salt thereof in the preparation of a drug for inhibiting osteosarcoma and/or chondrosarcoma.

Provided is the use of anlotinib or a pharmaceutically acceptable salt thereof as a potentiator of cisplatin in the inhibition of osteosarcoma and/or chondrosarcoma.

Further, the pharmaceutically acceptable salt of anlotinib is anlotinib hydrochloride.

Further, the drug inhibits the growth and/or metastasis of osteosarcoma and/or chondrosarcoma. Still further, the drug inhibits the lung metastasis of osteosarcoma and/or chondrosarcoma.

Further, the drug inhibits the recurrence of osteosarcoma and/or chondrosarcoma.

Further, the metastasis of osteosarcoma and/or chondrosarcoma is migration and/or invasion.

Further, the osteosarcoma is primary osteosarcoma and/or secondary osteosarcoma.

Further, the osteosarcoma is osteoblastic type and/or osteolytic type osteosarcoma.

Further, the osteosarcoma is osteoblastic osteosarcoma, chondroblastic osteosarcoma and/or fibroblastic osteosarcoma. Still further, the osteosarcoma is chondroblastic osteosarcoma.

Further, the chondrosarcoma is dedifferentiated chondrosarcoma and/or well-differentiated chondrosarcoma.

Further, the osteosarcoma is advanced and/or metastatic osteosarcoma, and the chondrosarcoma is advanced and/or metastatic chondrosarcoma.

Further, a subject with osteosarcoma and/or chondrosarcoma has been treated with chemotherapy and/or radiotherapy. Still further, a chemotherapeutic agent used in the chemotherapy received by the subject with osteosarcoma and/or chondrosarcoma includes methotrexate, ifosfamide, cisplatin, and doxorubicin. Still further, the disease progresses after the subject with osteosarcoma and/or chondrosarcoma has been treated with chemotherapy and/or radiotherapy.

Further, the drug comprises a therapeutically effective amount of anlotinib or a pharmaceutically acceptable salt thereof, and a pharmaceutically acceptable carrier.

The methods of treatment recited in the following paragraphs in the context of the invention are to be understood as pertaining to the recited substance(s) for use in methods for treating the recited diseases/conditions.

On the other hand, the present disclosure provides a method for treating osteosarcoma and/or chondrosarcoma, and the method includes administering a therapeutically effective amount of anlotinib or a pharmaceutically acceptable salt thereof to a patient in need of treatment.

Further, the present disclosure provides a method for inhibiting the growth and/or metastasis of osteosarcoma and/or chondrosarcoma, and the method includes administering a therapeutically effective amount of anlotinib or a pharmaceutically acceptable salt thereof to a patient in need of treatment. Still further, the present disclosure provides a method for inhibiting the lung metastasis of osteosarcoma and/or chondrosarcoma.

Further, the present disclosure provides a method for inhibiting the migration and/or invasion of osteosarcoma and/or chondrosarcoma, and the method includes administering a therapeutically effective amount of anlotinib or a pharmaceutically acceptable salt thereof to a patient in need of treatment.

Further, the present disclosure provides a method for treating primary osteosarcoma and/or secondary osteosarcoma, and the method includes administering a therapeutically effective amount of anlotinib or a pharmaceutically acceptable salt thereof to a patient in need of treatment.

Further, the present disclosure provides a method for treating osteogenic osteosarcoma and/or osteolytic osteosarcoma, and the method includes administering a therapeutically effective amount of anlotinib or a pharmaceutically acceptable salt thereof to a patient in need of treatment.

Further, the present disclosure provides a method for treating osteoblastic osteosarcoma, chondroblastic osteosarcoma and/or fibroblastic osteosarcoma, and the method includes administering a therapeutically effective amount of anlotinib or a pharmaceutically acceptable salt thereof to a patient in need of treatment.

Further, the present disclosure provides a method for treating dedifferentiated chondrosarcoma and/or well-differentiated chondrosarcoma, and the method includes administering a therapeutically effective amount of anlotinib or a pharmaceutically acceptable salt thereof to a patient in need of treatment.

Further, the present disclosure provides a method for treating advanced and/or metastatic osteosarcoma and/or chondrosarcoma, and the method includes administering a therapeutically effective amount of anlotinib or a pharmaceutically acceptable salt thereof to a patient in need of treatment.

Further, the present disclosure provides a method for preventing and/or treating the recurrence of osteosarcoma and/or chondrosarcoma, and the method includes administering a therapeutically effective amount of anlotinib or a pharmaceutically acceptable salt thereof to a patient in need of treatment.

Further, the present disclosure provides a method for treating a subject with osteosarcoma and/or chondrosarcoma that has been treated with chemotherapy and/or radiotherapy. Still further, the chemotherapeutic agent used in the chemotherapy received by the subject with osteosarcoma and/or chondrosarcoma includes methotrexate, ifosfamide, cisplatin, and doxorubicin. Still further, the disease progresses after the subject with osteosarcoma and/or chondrosarcoma has been treated with chemotherapy and/or radiotherapy.

Anlotinib may be administered in the form of a free base thereof, or in the form of a salt, or a hydrate thereof.

For example, a pharmaceutically acceptable salt of anlotinib is within the scope of the present disclosure, and said salt may be produced from different organic acids and inorganic acids according to methods well known in the art.

In some specific embodiments of the present disclosure, anlotinib is administered in the form of the hydrochloride salt thereof. In some specific embodiments, anlotinib is administered in the form of the monohydrochloride or dihydrochloride salt thereof. In some specific embodiments, anlotinib is administered in a crystalline form of the hydrochloride salt thereof. In some specific embodiments, anlotinib is administered in a crystalline form of the dihydrochloride salt of anlotinib.

Anlotinib or a pharmaceutically acceptable salt thereof may be administered via a variety of routes and said routes include, but are not limited to: oral administration, parenteral administration, intraperitoneal administration, intravenous administration, intra-arterial administration, transdermal administration, sublingual administration, intramuscular administration, rectal administration, transbuccal administration, intranasal administration, inhalational administration, vaginal administration, intraocular administration, topical administration, subcutaneous administration, intra-adipose administration, intraarticular administration, and intrathecal administration. In some specific embodiments, the drug is administered by oral administration, and the specific dosage forms include, but are not limited to, tablets, capsules, pulvis, granules, dropping pills, pastes, powders, and the like, preferably tablets and capsules. Among them, the tablets may be common tablets, dispersible tablets, effervescent tablets, sustained-release tablets, controlled-release tablets or enteric coated tablets, and the capsules may be common capsules, sustained-release capsules, controlled-release capsules or enteric coated capsules. The oral preparation may be prepared by a conventional method using a pharmaceutically acceptable carrier well known in the art. The pharmaceutically acceptable carrier includes, but is not limited to, a filler, an absorbent, a wetting agent, an binder, a disintegrant, a lubricant, and the like. The filler includes starch, lactose, mannitol, microcrystalline cellulose, and the like; the absorbent includes, but is not limited to, calcium sulfate, calcium hydrogen phosphate, calcium carbonate, and the like; the wetting agent includes water, ethanol, and the like; the binder includes, but is not limited to, hydroxypropyl methyl cellulose, povidone, microcrystalline cellulose, and the like; the disintegrant includes, but is not limited to, croscarmellose sodium, crospovidone, a surfactant, low-substituted hydroxypropyl cellulose, and the like; and the lubricant includes, but is not limited to, magnesium stearate, talc, polyethylene glycol, sodium lauryl sulfate, micronized silica gel, talc, and the like. The pharmaceutical excipient also includes a colorant, a sweetener, and the like.

In some specific embodiments of the present disclosure, the daily dose administered to a patient may be from 2 mg to 20 mg; in some specific embodiments, the daily dose administered to a patient may be from 5 mg to 20 mg; in some specific embodiments, the daily dose administered to a patient may be from 10 mg to 16 mg; in some specific embodiments of the present disclosure, the daily dose administered to a patient may be from 10 mg to 14 mg; and in some specific embodiments, the daily dose administered to a patient may be 8 mg, 10 mg, 12 mg, 14 mg, or 16 mg.

In the above treatment methods, anlotinib or a pharmaceutically acceptable salt thereof may be administered one or more times daily in a single dose or multiple doses. In some specific embodiments of the present disclosure, anlotinib or a pharmaceutically acceptable salt thereof is administered once a day.

The amount of anlotinib or a pharmaceutically acceptable salt thereof administered may be determined based on the severity of the disease, the disease response, any treatment-related toxicity, and the age and health status of the patient. Preferably, anlotinib or a pharmaceutically acceptable salt thereof is administered by means of intermittent administration, said intermittent administration includes an administration period and an off period, and anlotinib or a pharmaceutically acceptable salt thereof may be administered one or more times per day during the administration period. For example, anlotinib or a pharmaceutically acceptable salt thereof is administered daily during the administration period, the administration is then stopped for a period of time during the off period, followed by the administration period, then followed by the off period, and the above procedures may be repeated multiple times in this way. Among these, the ratio of the administration period to the off period in terms of days is 2 : 0.5 to 5, preferably 2 : 0.5 to 3, further preferably 2 : 0.5 to 2, and more preferably 2 : 0.5 to 1.

In some specific embodiments, the administration is continued for 2 weeks and discontinued for 2 weeks. In some specific embodiments, the drug is administered once a day for 14 consecutive days, followed by 14 days off, then the drug is administered once a day for 14 consecutive days, followed by 14 days off, so that the intermittent administration may be repeated multiple times in a manner where the administration lasts for 14 consecutive days followed by 14 days off.

In some specific embodiments, the administration is continued for 2 weeks and discontinued for 1 week. In some specific embodiments, the drug is administered once a day for 14 consecutive days, followed by 7 days off; then the drug is administered once a day for 14 consecutive days, followed by 7 days off, so that the intermittent administration may be repeated multiple times in a manner where the administration lasts for 14 consecutive days followed by 7 days off.

In some specific embodiments, the administration is continued for 5 days and discontinued for 2 days. In some specific embodiments, the drug is administered once a day for 5 consecutive days, followed by 2 days off; then the drug is administered once a day for 5 consecutive days, followed by 2 days off, so that the intermittent administration may be repeated multiple times in a manner where the administration lasts for 5 consecutive days followed by 2 days off.

In some embodiments, anlotinib or a pharmaceutically acceptable salt thereof is administered alone as the sole active ingredient to a patient with osteosarcoma or chondrosarcoma. In some embodiments, anlotinib or a pharmaceutically acceptable salt thereof is administered to a patient with osteosarcoma or chondrosarcoma together with other antitumor drugs simultaneously or sequentially. In some embodiments, other antitumor drugs include, but are not limited to, an alkylating agent, a platinum complex, a fluoropyrimidine derivative, camptothecin and camptothecin analogs, an anthraquinon-based antitumor antibiotic, and taxanes. In some embodiments, other antitumor drug is cisplatin.

Herein disclosed is a pharmaceutical composition for treating osteosarcoma and/or chondrosarcoma, comprising anlotinib or a pharmaceutically acceptable salt thereof and at least one pharmaceutically acceptable carrier. Herein disclosed is a kit comprising (a) a pharmaceutical composition comprising at least one unit dose of anlotinib or a pharmaceutically acceptable salt thereof and (b) an instruction for treating osteosarcoma and/or chondrosarcoma.

The advantages of the present disclosure are as follows.
1. It has been discovered for the first time that anlotinib may inhibit the growth and metastasis of osteosarcoma and/or chondrosarcoma. Anlotinib is capable of significantly inhibiting the growth of the osteosarcoma cell lines 143B, U2OS, MG63 and SJSA, inducing cycle arrest in said cell lines, and inhibiting the migration and invasion of osteosarcoma cell lines in the meantime as well
2. It has been discovered for the first time that anlotinib is capable of enhancing the killing effect of the chemotherapeutic drug cisplatin on osteosarcoma cells.
3. It has been discovered for the first time that anlotinib may be used for treating osteosarcoma and/or chondrosarcoma.

Unless otherwise specified, for the purposes of the present application, the following terms used in this specification and the claims shall have the following meanings.

A "patient" refers to a mammal, preferably a human.

The term "pharmaceutically acceptable" means that a substance may be used to prepare a pharmaceutical composition. Said pharmaceutical composition is generally safe, non-toxic, neither biologically undesirable nor otherwise undesirable, and acceptable for human pharmaceutical use.

A "pharmaceutically acceptable salt" includes, but is not limited to, the acid addition salts formed with inorganic acids such as hydrochloric acid, hydrobromic acid, sulfuric acid, nitric acid, phosphoric acid, and the like; or the acid addition salts formed with organic acids such as acetic acid, trifluoroacetic acid, propionic acid, hexanoic acid, heptanoic acid, cyclopentanepropionic acid, glycolic acid, pyruvic acid, lactic acid, malonic acid, succinic acid, malic acid, maleic acid, fumaric acid, tartaric acid, citric acid, benzoic acid, cinnamic acid, mandelic acid, methanesulfonic acid, ethanesulfonic acid, 1,2-ethionic acid, 2-isethionic acid, benzenesulfonic acid, p-chlorobenzenesulfonic acid, p-toluenesulfonic acid, 3-phenylpropionic acid, trimethylacetic acid, t-butylacetic acid, dodecyl sulfuric acid, gluconic acid, glutamic acid, hydroxynaphthoic acid, salicylic acid, stearic acid, and the like.

A "therapeutically effective amount" refers to an amount of a compound that, when administered to a human for the treatment of a disease, is sufficient to achieve the control of said disease.

"Treatment" refers to any administration of a therapeutically effective amount of a compound, and includes:
(1) inhibiting a disease in a human body experiencing or exhibiting the pathology or symptomatology of the disease (i.e., preventing further development of the pathology and/or symptomatology), or
(2) ameliorating a disease in a human body experiencing or exhibiting the pathology or symptomatology of the disease (i.e., reversing the pathology and/or symptomatology).

"CR" refers to complete response, specifically means that the tumor target lesion disappears, no new lesion appears, and the tumor marker is normal and maintained for at least 4 weeks.

"PR" refers to partial response, specifically means that the sum of the diameters of the tumor target lesions is reduced by 30% or more from the baseline level and is maintained for at least 4 weeks.

"PD" refers to progressive disease, specifically means that the sum of the diameters of the tumor target lesions increases by 20% or more from the baseline level.

"SD" refers to stable disease, specifically means that the reduction degree of the tumor target lesion(s) does not reach the PR level, the increase degree also does not reach the PD level, and the SD level is between the PR level and the PD level.
"qd" refers to taking medicine once a day.

"Advanced" includes "locally advanced".

### BRIEF DESCRIPTION OF THE DRAWINGS

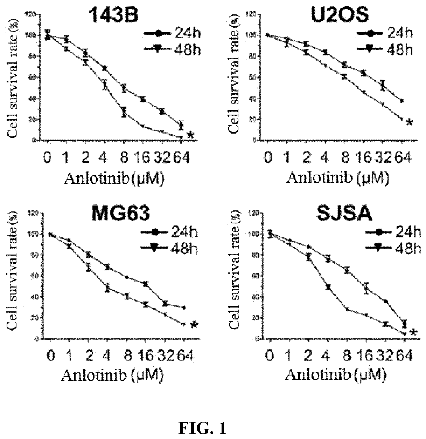

Figure 1: Anlotinib inhibits the proliferation of osteosarcoma cells.

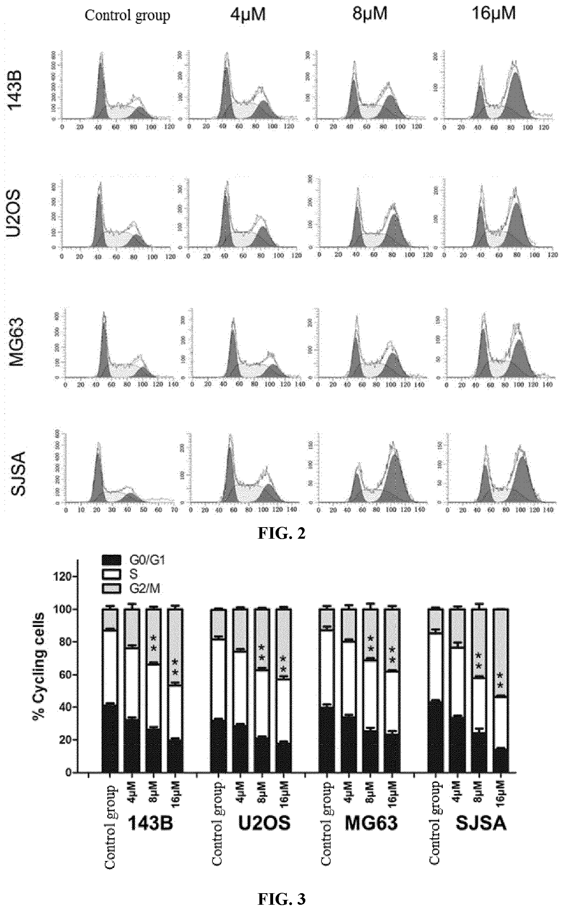

Figure 2: Anlotinib induces the cell cycle arrest of osteosarcoma cells.
Figure 3: Anlotinib induces the cell cycle arrest of osteosarcoma cells.

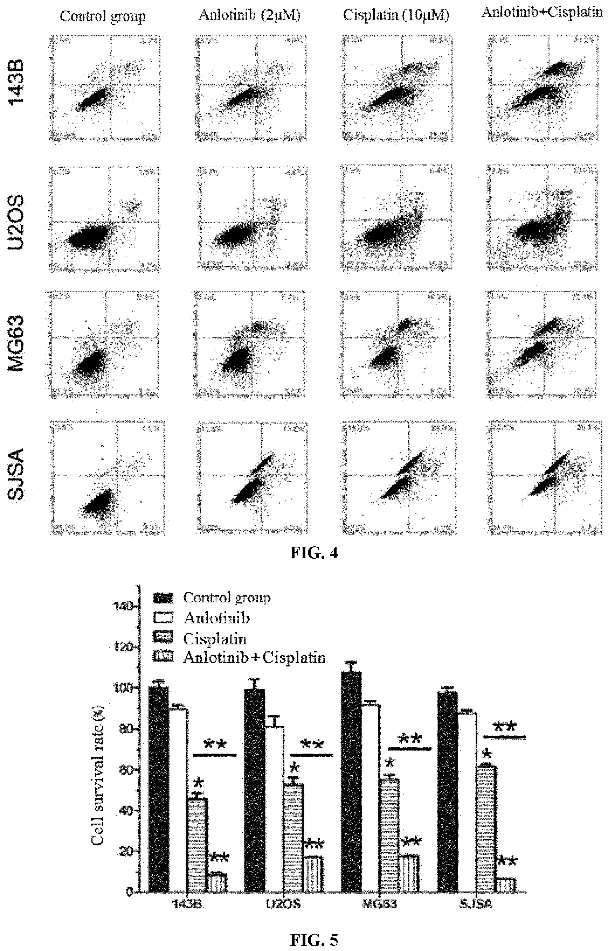

Figure 4: Anlotinib enhances the apoptosis of osteosarcoma cells induced by cisplatin (DDP).
Figure 5: Anlotinib enhances the apoptosis of osteosarcoma cells induced by cisplatin (DDP).

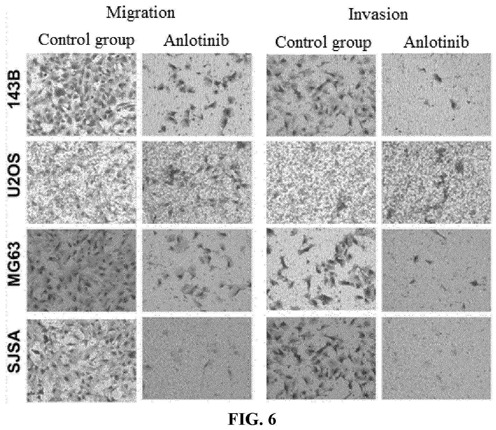

Figure 6: Anlotinib inhibits the migration and invasion of osteosarcoma cells.

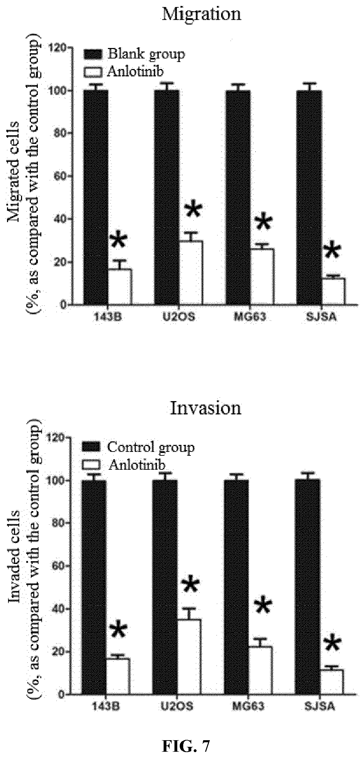

Figure 7: Anlotinib inhibits the migration and invasion of osteosarcoma cells.

### DETAILED DESCRIPTION

Hereinafter, the specific embodiments provided by the present disclosure will be described in detail with reference to Examples.

### Example 1

### 1. Materials and methods

1.1 Materials: Human osteosarcoma cell lines 143B, U2OS, MG63 and SJSA were purchased from the American Type Culture Collection (ATCC). Anlotinib (a novel tyrosine kinase inhibitor) was obtained from CHIA TAI TIANQING PHARMACEUTICAL GROUP CO., LTD. Anlotinib was dissolved in dimethyl sulfoxide and formulated into a working solution with a concentration of 16 mmol/L as the stock solution, which was placed in a refrigerator at -20°C for subsequent use and was adjusted to a desired concentration with DMEM culture medium containing serum prior to use.

Experimental reagents and instruments: DMEM high-glucose medium, fetal bovine serum (Thermo, USA); cleaved-Caspase3, an antibody against p-PARP, an antibody against GAPDH, a horseradish peroxidase-labeled secondary antibody (Sixin Biotechnology Co., Ltd.); dimethyl sulfoxide (Sigma, USA); 0.02% EDTA + 0.25% trypsin (Miltenyi Biotec Co., Germany); thermostatic incubator (Shanghai Rongyan Instrument Co., Ltd.); flow cytometer (Becton Dickinson Co., USA); multifunctional microplate reader (Molecular Devices Co., USA).

### 1.2 Experimental methods

1.2.1 Cell culture: Osteosarcoma cell lines 143B, U2OS, MG63 and SJSA were cultured in DMEM complete culture medium (containing fetal bovine serum with a volume fraction of 10%, 0.1 g/L streptomycin, and 100 U/mL penicillin), and was incubated in a thermostatic incubator containing 5% CO₂ (volume fraction) at 37°C. When about 85% cell confluence was reached, the cells were detached by using a mixed cell detachment solution containing 0.02% EDTA and 0.25% trypsin, and then the cells were collected, centrifuged at 1000 r/min for 3 min, and subcultured.

1.2.2 Detection of cell proliferation by cck-8: 143B cells, U2OS cells, MG63 cells and SJSA cells in logarithmic growth phase were collected, rinsed with PBS, counted, and dispensed into separate tubes. The cells were seeded into a 96-well plate (3000 cells/well) and cultured for 24 h to allow for cell attachment. Anlotinib with different amount-of-substance concentrations (0 µM, 1 µM, 2 µM, 4 µM, 8 µM, 16 µM, 32 µM, and 64 µM) were then added, the mixture was incubated for 24 h, and then the detection was carried out. 100 µL of cck-8 reagent diluted 10-fold with DMEM culture medium was added into each well and the resulting mixture was incubated at 37°C for 45 minutes. The absorbance value of the resultant in each well at 450 nm was detected by using a microplate reader.

1.2.3 Plate cloning experiment: 143B cells, U2OS cells, MG63 cells and SJSA cells were treated with 1 µM Anlotinib, and the supernatant was aspirated. The cells were fixed with 4% paraformaldehyde for 15 min, rinsed 3 times with PBS, stained with 0.1% crystal violet for 10 min, rinsed twice with PBS, and photographed after air drying.

1.2.4 Detection of cell cycle by flow cytometer: 143B cells, U2OS cells, MG63 cells and SJSA cells were added to the liquid culture media containing different amount-of-substance concentrations of Anlotinib (0 µM, 1 µM, 2 µM, and 4 µM) for cultivation. The cells were collected after 24 h, centrifuged at 1000 r/min for 1 min, rinsed with PBS, and fixed with 70% ethanol. The PI staining solution of cell cycle analysis kit was added, the mixture was incubated in the dark at room temperature for 15 min, and the detection was carried out by using a flow cytometer. Images were analyzed by using ModFit software. The experiment was repeated 3 times.

1.3.5 Detection of cell apoptosis by flow cytometer: 143B cells, U2OS cells, MG63 cells and SJSA cells were added to the liquid culture media containing 2 µM Anlotinib (with or without 10 µM cisplatin (DDP)) for cultivation. The cells were collected after 24 h, centrifuged at 1000 r/min for 1 min, and rinsed with PBS. Afterwards, an Annexin-V-FITC/PI Cell Apoptosis Detection Kit was used to detect the apoptosis of cells. The cells were added to 100 µL of 1× Binding buffer and resuspended, 5 µL of AnnexinV-FITC and 2.5 µL of PI staining reagent were added, and the mixture was shaken and mixed evenly in the dark. The resultant was reacted at room temperature for 15 min, 300 µL of 1× Binding buffer was then added, and the resulting mixture was mixed evenly and detected by a flow cytometer. The experiment was repeated 3 times.

1.3.6 Experimental analysis of Transwell cell migration and invasion: Cells were seeded at the upper end of the Transwell chamber (5 × 10⁴ cells/well). 143B cells, U2OS cells, MG63 cells and SJSA cells were treated with 1 µM Anlotinib. A culture medium containing 2% serum was added to the upper end of the Transwell chamber (4 hours of pretreatment with 10% matrigel was required in the invasion experiment), and a culture medium containing 10% serum was added to the lower end. After 24 hours, the cells were fixed with 4% paraformaldehyde, stained with crystal violet, and photographed under microscope.

1.4 Main observation indexes. Anlotinib was capable of exerting significant inhibitory effect on the growth and metastasis of osteosarcoma cells, while being capable of enhancing the killing effects of chemotherapeutic drugs on osteosarcoma cells.

### 2. Results

### 2.1 Anlotinib inhibited the proliferation of osteosarcoma cells

In order to evaluate the anti-proliferation effects of Anlotinib on osteosarcoma cell lines 143B, U2OS, MG63 and SJSA, the osteosarcoma cell lines were treated with Anlotinib with a concentration of 0 µM, 1 µM, 2 µM or 4 µM for 24 hours or 48 hours. The results indicated that, the proliferation rate of cells decreased with the increase of the concentration of Anlotinib, and Anlotinib also had significant inhibitory effect on the proliferation of the osteosarcoma cell lines under same concentration and different duration of drug action. The above results indicated that Anlotinib had significant inhibitory effect on the proliferation of the osteosarcoma cell lines and the inhibitory effect appeared as time- and concentration-dependent.

**Table 1**

| Cell lines | 143B | U2OS | MG63 | SJSA |
|---|---|---|---|---|
| IC50 (24 h) | 20.81 | 41.28 | 30.11 | 25.66 |
| IC50 (48 h) | 7.95 | 25.92 | 14.84 | 10.22 |

### 2.2 Anlotinib caused cell cycle arrest in osteosarcoma cells

In order to study the mechanism by which Anlotinib inhibited the proliferation of osteosarcoma cells, cell cycle detection was conducted using the osteosarcoma cell lines 143B, U2OS, MG63 and SJSA as target cells. The results indicated that, after the treatment with Anlotinib, G2/M cycle arrest occurred in cells, which was accompanied by a decrease of cells in G1 phase at the same time.

### 2.3 Anlotinib enhanced the apoptosis of osteosarcoma cells caused by cisplatin (DDP)

Next, the inventors explored the chemosensitization effect of Anlotinib on osteosarcoma cell lines, 2 µM Anlotinib was used, and the classic Annexin-V-FITC/PI cell apoptosis assay was adopted. The results indicated that Anlotinib was capable of significantly enhancing the apoptosis of osteosarcoma cell lines caused by cisplatin.

### 2.4 Anlotinib inhibited the migration and invasion of osteosarcoma cells

In order to study the effect of Anlotinib on the migration and invasion of osteosarcoma cell lines, the inventors adopted Transwell analysis. The results indicated that Anlotinib greatly inhibited the migration and invasion of osteosarcoma cell lines after 24 hours of treatment with Anlotinib.

### 3 Conclusions

Anlotinib was capable of significantly inhibiting the growth of osteosarcoma cell lines 143B, U2OS, MG63 and SJSA, inducing the cell cycle arrest thereof, and inhibiting the migration and invasion of osteosarcoma cell lines in the meantime as well. Further research indicated that Anlotinib was also capable of enhancing the killing effect of the chemotherapeutic drug cisplatin on osteosarcoma cells.

### Example 2

### Capsules containing 1-[[[4-(4-fluoro-2-methyl-1H-indol-5-yl)oxy-6-methoxyquinolin-7-yl]oxy]methyl] cyclopropylamine dihydrochloride (Anlotinib dihydrochloride)

| Name of raw materials and excipients | Amount (1000 capsules) |
|---|---|
| Anlotinib dihydrochloride | 14.16 g (equivalent to 12 g of Anlotinib) |
| Mannitol | 89 g |
| Microcrystalline cellulose | 138.4 g |
| Hydroxypropylcellulose | 5.9 g |
| Magnesium stearate | 0.99 g |

Anlotinib dihydrochloride was pulverized, allowed to pass through an 80-mesh sieve, and then mixed with mannitol and hydroxypropylcellulose evenly. Next, the prescribed amount of microcrystalline cellulose was added and mixed evenly, and the mixture was allowed to pass through a 0.8-mm sieve. Finally, the prescribed amount of magnesium stearate was added and mixed evenly, and the resulting mixture was filled into capsules.

Capsules having other contents of anlotinib dihydrochloride could be prepared and obtained with reference to the same proportion and prescription as described above.

### Example 3

A 22-year-old male patient came for consultation, the post-operative pathological findings suggested osteosarcoma of the left proximal humerus, and the chest CT showed lung metastasis.

The patient experienced pain and swelling in the left shoulder with no obvious predisposing cause and the symptoms became worse at night. After the consultation, MR images of the left humerus showed malignant bone tumor in the middle and upper part of the left humerus. Shoulder girdle amputation was performed. The post-operative pathological findings suggested (left) proximal humerus osteosarcoma with invasion of the shaft of humerus and the surrounding striated muscle tissue and without involvement of the muscle and skin tissues at the amputated end caused by the surgery, and no metastatic tumor was found in lymph nodes. The chest CT showed nodular shadows in both lungs, and re-examination was recommended. The patient was administered with methotrexate and epirubicin (1 cycle) for one month from the day of consultation, and the dosage regimen was switched to epirubicin and cisplatin chemotherapy (1 cycle) after one month due to the abnormal liver function. Subsequently, the chest CT showed multiple nodules in lungs, which was considered as metastasis, and the dosage regimen was switched to epirubicin, cisplatin and ifosfamide chemotherapy (4 cycles). The optimum therapeutic effect was SD.

Nine months after the consultation, the patient was treated with anlotinib dihydrochloride capsule (12 mg) once a day via oral administration (2 weeks of continuous administration and 1 week of withdrawal being considered as one treatment cycle).

After the patient was administered for 2 cycles, the chest, abdomen and pelvis CT showed that the therapeutic effect was evaluated as SD (small) based on RECIST1.1, and the total size of the target lesions was 40 mm, which did not change much as compared with the baseline (41 mm). After 3 cycles of administration, the chest, abdomen and pelvis CT showed that the total size of the target lesions was 37 mm, which was slightly reduced as compared with the previous one. After 5 cycles of administration, the chest, abdomen and pelvis CT showed that the lesions were reduced as compared with the previous ones and the total size of the target lesions was 34 mm. After 8 cycles of administration, the chest, abdomen and pelvis CT showed that the total size of the target lesions was 30 mm. After 12 cycles of administration, the chest and abdomen CT showed that the target lesions were further reduced and the therapeutic effect was evaluated as PR based on RECIST1.1, and the total size of the target lesions was 27 mm. After 15 cycles of administration, the chest and abdomen CT showed that the total size of the target lesions was 27 mm, and the therapeutic effect was evaluated as PR. After 18 cycles of administration, the chest and abdomen CT showed that the target lesions were further reduced, and the total size of the target lesions was 20 mm. After 21 cycles of administration, the chest and abdomen CT showed that the total size of the target lesions was 24 mm, which was slightly increased as compared with the previous one, and the therapeutic effect was evaluated as PR.

### Example 4

A 38-year-old female patient was diagnosed with chondrosarcoma of the cervico-thoracic spine by CT-guided puncture. PET-CT showed multiple metastases to both lungs, right adrenal gland and pancreatic tail.

The patient experienced pain in the right back, shoulders and right upper limb accompanied by numbness and swelling of the right upper limb with no obvious predisposing cause. The patient came for consultation, and the cervical MRI showed space-occupying lesions in the right upper mediastinum and the paraspinal space. The puncture was conducted under the guidance of CT, and the pathological findings showed consistency with those of dedifferentiated chondrosarcoma. Subsequently, the following surgeries were performed, i.e., the excision of T1-2 adnexas and the tumor in intervertebral foramen via posterior approach; the fixation of C6, 7-T2; the VATS-assisted right thoraco-axillary incision; and the resection of posterior mediastinal tumor. The post-operative pathologies all suggested chondrosarcoma. Afterwards, a local radiotherapy targeting at cervical spine and thoracic spine were performed at 50 Gy/25 f. Regional pain and discomfort appeared after 16 months, and the patient received gemcitabine and Endostar chemotherapy (3 cycles) and biological therapy (2 cycles). After 2 months, a neck MRI re-examination showed recurrence of the lesion, and the recurrent lesion was treated by a stereotactic accelerator. After 6 months, PET-CT re-examination showed: tumor recurrence and metastasis in a part of the spinal canal and intervertebral foramen; the invasion of some adjacent bones; multiple metastatic tumors in both lungs, right pleura, hilum of the left lung, right adrenal gland and pancreatic tail; and the invasion of the left psoas major muscle which could not be excluded. Subsequently, the treatment of the lesions in vertebral body was performed by using a stereotactic accelerator. After the treatment, the pain in the neck and shoulders of the patient was not alleviated, and the patient was orally administered with Mescontin (30 mg) twice a day.

After one month, the patient was treated with anlotinib dihydrochloride capsule (12 mg) once a day via oral administration (2 weeks of continuous administration and 1 week of withdrawal being considered as one treatment cycle).

After the patient was administered for 2 cycles, CT showed scattered multiple nodules and masses in both lungs and pleura, some of which were similar to the previous ones, and some of which were slightly reduced as compared with the previous ones. The mass in the right lower pleura invaded the right diaphragm and the chest wall, had a less clear boundary with the right adrenal gland, and was slightly reduced as compared with the previous one. In the lower boundary of the scanning field, the low-density mass in the left psoas muscle was reduced as compared with the previous one. The total size of the target lesions was 148 mm, which was reduced by 25 mm as compared with the baseline (173 mm), and the therapeutic effect was evaluated as SD (small) based on RECIST1.1. After the patient was administered for 4 cycles, CT showed that the total size of the target lesions was 139 mm, which was slightly reduced as compared with the previous one. After the patient was administered for 6 cycles, CT showed that the total size of the target lesions was 126 mm, which was reduced by 13 mm as compared with the previous one. After the patient was administered for 7 cycles, CT showed that the total size of the target lesions was 131 mm, which was slightly increased as compared with the previous one. After the patient was administered for 11 cycles, CT showed that the total size of the target lesions was 127 mm, which was slightly reduced as compared with the previous one. After the patient was administered for 14 cycles, CT showed that the total size of the target lesions was 129 mm, which did not change much as compared with the previous one. After the patient was administered for 17 cycles, CT showed that the total size of the target lesions was 131 mm, which did not change much as compared with the previous one. After the patient was administered for 21 cycles, CT showed that the total size of the target lesions was 138 mm, which was slightly increased as compared with the previous one. After the patient was administered for 25 cycles, CT showed that the total size of the target lesions was 129 mm, which was slightly reduced as compared with the previous one.

### Example 5

A 27-year-old female patient was diagnosed with pelvic chondrosarcoma by CT and the post-operative pathology, and the lesion progressed after multiple treatments.

The patient found a mass in the lower abdomen and came for consultation. CT showed that an irregular huge mass shadow with mixed density could be seen in the pelvic cavity, the uterus and the front lower part of the bladder, and the mass had a maximum cross-section of 16 cm × 11 cm × 13 cm. Surgical resection was performed subsequently. Post-operative pathology: well-differentiated chondrosarcoma. The patient did not receive postoperative chemoradiotherapy. A re-examination performed 15 months after the consultation revealed the recurrence of the pelvic mass. Surgical treatment was performed 6 months later, and the tumor was unresectable due to the extensive adhesions found during the surgery. Post-operative pathology: well-differentiated chondrosarcoma. Subsequently, the patient underwent an arterial infusion chemotherapy for the pelvic tumor, and was administered with pirarubicin (60 mg), cisplatin (60 mg) and interleukin-2 (2 million U). After one month, the patient continuously underwent the arterial infusion chemotherapy for the abdominal and pelvic tumor, and was administered with pirarubicin (60 mg), cisplatin (60 mg) and interleukin-2 (2 million U). The patient experienced worse lumbosacral pain which radiated to the left thigh, and the pain was such severe that the patient was unable to sit up and walk on her feet. CT showed the progression of the abdominal and pelvic mass.

After 2 months, the patient was treated with anlotinib dihydrochloride capsule (12 mg) once a day via oral administration (2 weeks of continuous administration and 1 week of withdrawal being considered as one treatment cycle).

After 2 cycles of administration, a CT scan was performed and the results showed that the total size of the target lesions was 210 mm, which was unchanged from the baseline (210 mm), and the therapeutic effect was evaluated as SD. After 4 cycles of administration, the CT re-examination showed that the total size of the target lesions was 210 mm, which was unchanged as compared with the previous one. After 6 cycles of administration, the total size of the target lesions was 220 mm, which was increased by 10 mm as compared with the previous one. After 8 cycles of administration, the CT re-examination showed that the total size of the target lesions was 240 mm, which was increased by 20 mm as compared with the previous one. After 11 cycles of administration, the CT re-examination showed that the total size of the target lesions was 240 mm, which was unchanged as compared with the previous one. After 14 cycles of administration, the CT re-examination showed that the total size of the target lesions was 240 mm, which was unchanged as compared with the previous one.

### Example 6

A 25-year-old male patient underwent the surgical removal of the osteosarcoma lesion in the right proximal humerus and the humeral head prosthesis replacement on April 27, 2013. The post-operative pathological findings suggested osteosarcoma (chondroblastic type) of the right proximal humerus which involved the bone marrow cavity and invaded the bone cortex and the soft tissue, focal necrosis and fat necrosis could be seen in the tumor, and no infiltration of tumor tissue was seen at the epiphyseal and inferior incisal margin. Doxorubicin and ifosfamide were administered after the surgery. The patient underwent 4 courses of chemotherapy, during which severe bone marrow suppression occurred. Bone marrow support therapy was given during the intermission of chemotherapy until the end of chemotherapy in August 2013. During the chemotherapy, the whole-body PET/CT re-examination performed in June 2013 showed the following conditions. 1. After the surgery of the osteosarcoma of the right humerus and the chemotherapy, a stripe-shaped shadow with slightly increased metabolism appeared around the artificially implanted bone in the right upper arm. 2. There were enlarged lymph nodes in the right axilla with slight increase in metabolism. The patient was treated by being administered with CIK cells via infusion after the completion of the chemotherapy. At the end of 2015, the patient experienced pain in the upper part of the left humerus, no obvious mass was seen, and the patient suffered from the recurrent pain. MRI performed in May 2016 showed an abnormal change in the middle and upper part of the left humerus, which was an infectious lesion or a swelling and painful lesion. PET/CT performed on May 12, 2016 showed the following conditions. 1. There was no sign of tumor recurrence in the right humerus after the surgery of the osteosarcoma of the right humerus and the comprehensive treatment, and there was a stripe-shaped hypermetabolic lesion in the upper part of the left humerus. 2. There was no sign of lymph node metastasis in the bilateral armpits. 3. There were slightly hypermetabolic and enlarged lymph nodes in the bilateral armpits, a biopsy of the mass in the left humerus was performed subsequently and the pathological findings suggested round-cell tumor which was more likely to be diagnosed as chondroblastic osteosarcoma. On June 7, 2016 and July 1, 2016, the patient underwent 2 courses of doxorubicin and ifosfamide chemotherapy and the process went well. The MRI re-examination performed after the chemotherapy showed that the tumor was slightly larger than before. Microwave inactivation of the tumor of the left proximal humerus and the bone graft internal fixation was performed on July 22, 2016. The post-operative pathological findings showed consistency with the pathological changes of the recurrence of osteosarcoma, and it could be seen that the muscle tissues adjacent to the tumor were involved. High doses of methotrexate were administered on August 17, September 8, October 8, October 31, November 28 and December 21 in 2016. The patient underwent 6 courses of chemotherapy and the process went well. The bone marrow suppression was rated as Grade I to II after the completion of the chemotherapy. Bilateral lung metastasis was found in November 2017, the disease progressed, and the chemotherapy was not continued. The CT re-examination performed on October 20, 2018 showed that there were scattered multiple nodular shadows with different sizes in both lungs, said nodular shadows were increased in number and size as compared with the previous ones, and the disease progressed.

The patient was treated with anlotinib dihydrochloride capsule (12 mg) once a day via oral administration (2 weeks of continuous administration and 1 week of withdrawal being considered as one treatment cycle) from November 10, 2018. On December 21, 2018, the patient had received 2 cycles of treatment, and a plain CT scan showed multiple nodules and masses in both lungs, in which the largest lesion (3.2 cm) was located in the middle lobe of the right lung. The therapeutic effect was evaluated as SD (small) based on RECIST1.1. The total size of the target lesions was 53.5 mm, which was reduced by 5.5 mm as compared with the baseline. The patient was receiving continuous treatment at present.

### Example 7

An 18-year-old female patient underwent the resection of the lesion on November 7, 2016. On November 15, 2016, the pathological findings suggested osteosarcoma of the right tibia. PORT implantation was performed under general anesthesia on November 29, 2016, and the postoperative systemic intravenous adjuvant chemotherapy was performed. Doxorubicin and cisplatin (1 cycle of chemotherapy) were administered on December 1, 2016, and methotrexate (2 cycles of chemotherapy) was administered from December 16, 2016 to January 5, 2017. The CT re-examination performed on January 19, 2017 showed that the lesion in the right tibia was slightly larger than before. Ifosfamide (1 cycle of chemotherapy) was administered on January 19, 2017, and the chemotherapy went well with no obvious gastrointestinal reaction and bone marrow suppression. The segmental resection of the tumor of the right proximal tibia, the j oint replacement and the transposition of gastrocnemius muscle flap were performed under general anesthesia on February 7, 2017. Intraoperative diagnosis: malignant osteosarcoma of the right proximal tibia. Post-operative pathology: (the incisal margin of the right tibial medullary cavity) small amount of broken bones and intramedullary adipose tissue. Pathology report of the tumor segment: (right proximal tibia) osteosarcoma, wherein the tumor invaded and penetrated the cartilage of the articular surface, extensively penetrated the cortical bone and invaded the surrounding soft tissue, and a tumor thrombus was seen in the vessel. Doxorubicin and cisplatin (1 course of chemotherapy) were administered on February 24, 2017, and the patient experienced leucocytopenia (Grade III) after the chemotherapy . Ifosfamide (1 course of chemotherapy) was administered on March 10, 2017, and the patient experienced leucocytopenia (Grade II) after the chemotherapy and did not experience obvious side effects resulting from the chemotherapy. Methotrexate was administered on March 31, 2017, and the patient experienced liver injury (Grade IV), which was ameliorated after the symptomatic treatment. Doxorubicin and cisplatin (1 course of chemotherapy) were administered on April 15, 2017. After the prophylaxis of vomiting, the patient experienced vomiting (Grade II), which was ameliorated after the symptomatic treatment. Ifosfamide chemotherapy was given on May 3, 2017, and the patient experienced leucocytopenia (Grade IV) after the chemotherapy. Methotrexate chemotherapy was given on May 20, 2017, and the patient experienced liver injury (Grade II) after the chemotherapy. Doxorubicin and cisplatin chemotherapy was given on June 4, 2017, ifosfamide chemotherapy was given on June 19, 2017, and the patient experienced leucocytopenia (Grade IV). Methotrexate chemotherapy was given on July 11, 2017, and the patient experienced liver injury (Grade I ). Doxorubicin and cisplatin chemotherapy was given on July 29, 2017, and the patient experienced leucocytopenia (Grade II). Ifosfamide chemotherapy was given on August 16, 2017, and then the patient experienced leucocytopenia (Grade IV). Methotrexate chemotherapy was given on September 6, 2017, and the patient experienced bone marrow suppression (Grade II). The CT re-examination performed on January 3, 2018 showed that the small nodules at the base of the lower lobe of the left lung were roughly similar to the previous nodules and the thin and small nodules in the dorsal segment of the lower lobe of the left lung were newly formed. The CT re-examination performed in October 2018 showed lung metastasis, pelvic metastasis and a mass behind the right femur, indicating the possibility of recurrence. A puncture biopsy of the mass of right femur was performed on October 30, 2018, and the pathological findings suggested high-grade spindle cell sarcoma with massive coagulative necrosis (in the soft tissue of the right lower femur).

The patient was treated with anlotinib dihydrochloride capsule (12 mg) once a day via oral administration (2 weeks of continuous administration and 1 week of withdrawal being considered as one treatment cycle) from November 16, 2018. The patient had received 2 cycles of treatment by December 26, 2018. CT showed mixed-density nodules in the lower lobe of the right lung, small nodules at the base of the lower lobe of the left lung which were roughly similar to the previous nodules, a mass shadow at the right pelvic wall which was accompanied by ossification and necrosis and was smaller than before. In addition, CT showed that the right tibia was roughly similar to the previous one. The therapeutic effect was evaluated as SD (small) based on RECIST1.1. The total size of the target lesions was 218 mm, which was reduced by 13 mm as compared with the baseline. The patient was receiving continuous treatment at present.

### Example 8

A 32-year-old female patient experienced a pain on the outer side of the right lower leg with no obvious predisposing cause for 6 months. The pain was persistent and unrelated to movements such as walking, and could not be alleviated after rest. The patient was neither diagnosed nor treated, and the pain was gradually aggravated and occurred at night. An X-ray film was taken and the film showed osteolytic damage at the proximal end of the right fibula. The patient went for consultation on December 1, 2017, and an incision biopsy of the tumor of the limb was performed on December 5. The pathological diagnosis on December 7 suggested mesenchymal non-small cell malignant tumor of the right proximal fibula, and osteosarcoma could not be excluded when combining the pathological diagnosis with the clinical imaging. On April 19, 2018, the post-operative pathological findings suggested osteosarcoma of the right proximal fibula accompanied by changes occurred after the chemotherapy, and the tumor necrosis rate was less than 90%.

Methotrexate chemotherapy was given for 1 day on December 12, 2017, February 7, 2018, May 4, 2018 and July 6, 2018, respectively. IFO chemotherapy (1 cycle) was given during the following time periods: from December 16, 2017 to December 30, 2017, from March 2, 2018 to March 7, 2018 (the therapeutic effect was evaluated as SD), from April 18, 2018 to April 22, 2018 (platelet reduction (Grade IV)), from June 14, 2018 to June 18, 2018 (the therapeutic effect was evaluated as SD), from August 30, 2018 to September 3, 2018 (the therapeutic effect was evaluated as PD). Cisplatin and amrubicin chemotherapy (1 cycle) was respectively given during the following time periods: from January 17, 2018 to January 20, 2018, from May 24, 2018 to May 27, 2018, and from July 20, 2018 to July 23, 2018 (the therapeutic effect was evaluated as SD). The comprehensive evaluation revealed that the first-line chemotherapy was tolerable for the patient at the beginning (from December 2017 to March 2018), the second-line chemotherapy was poorly tolerated at later period (after April 2018), the bone marrow suppression was obvious, and the condition of the patient progressed.

The medical history of the patient complied with the requirements of the clinical trial of anlotinib hydrochloride for the treatment of osteosarcoma as an indication. The patient was treated with anlotinib dihydrochloride capsule (12 mg) once a day via oral administration (2 weeks of continuous administration and 1 week of withdrawal being considered as one treatment cycle) from October 12, 2018. On November 23, 2018, the first tumor assessment was performed after the patient had been administered for 2 cycles. As compared with the baseline (37 mm) (the size of the target lesions in the middle lobe of the right lung was 24 mm, and the size of the target lesions in the lower lobe of the right lung was 13 mm), the size of the target lesions was reduced to 20 mm (the size of the target lesions in the middle lobe of the right lung was 10 mm, and the size of the target lesions in the lower lobe of the right lung was 10 mm). On January 3, 2019, the tumor assessment was performed after 4 cycles of administration. The total long diameter of the target lesions was 16 mm (the size of the target lesions in the middle lobe of the right lung was 10 mm, and the size of the target lesions in the lower lobe of the right lung was 6 mm). The patient was receiving continuous treatment at present.

Those described above are merely the preferred embodiments of the present disclosure.

## Claims

1. Anlotinib or a pharmaceutically acceptable salt thereof, for use in inhibiting osteosarcoma and/or chondrosarcoma in a patient in need of treatment.

2. Anlotinib or a pharmaceutically acceptable salt thereof, for use as a potentiator of cisplatin in the inhibition of osteosarcoma and/or chondrosarcoma in a patient in need of treatment.

3. Anlotinib or a pharmaceutically acceptable salt thereof for use according to claim 1 or 2, wherein the pharmaceutically acceptable salt of anlotinib is anlotinib hydrochloride, preferably dihydrochloride.

4. Anlotinib or a pharmaceutically acceptable salt thereof for use according to claim 1 or 2, wherein the drug inhibits growth and/or metastasis and/or recurrence of osteosarcoma and/or chondrosarcoma.

5. Anlotinib or a pharmaceutically acceptable salt thereof for use according to claim 4, wherein the metastasis of osteosarcoma and/or chondrosarcoma is migration and/or invasion.

6. Anlotinib or a pharmaceutically acceptable salt thereof for use according to claim 1 or 2, wherein the osteosarcoma is primary osteosarcoma and/or secondary osteosarcoma.

7. Anlotinib or a pharmaceutically acceptable salt thereof for use according to claim 1 or 2, wherein the osteosarcoma is osteogenic osteosarcoma and/or osteolytic osteosarcoma.

8. Anlotinib or a pharmaceutically acceptable salt thereof for use according to claim 1 or 2, wherein the osteosarcoma is osteoblastic osteosarcoma, chondroblastic osteosarcoma and/or fibroblastic osteosarcoma.

9. Anlotinib or a pharmaceutically acceptable salt thereof for use according to claim 1 or 2, wherein the chondrosarcoma is dedifferentiated chondrosarcoma and/or well-differentiated chondrosarcoma.

10. Anlotinib or a pharmaceutically acceptable salt thereof for use according to claim 1 or 2, wherein the osteosarcoma and/or chondrosarcoma is advanced and/or metastatic osteosarcoma and/or chondrosarcoma; preferably, the metastatic osteosarcoma and/or chondrosarcoma is an osteosarcoma and/or chondrosarcoma with lung metastasis.

11. Anlotinib or a pharmaceutically acceptable salt thereof for use according to claim 1 or 2, wherein a subject with the osteosarcoma and/or chondrosarcoma has been treated with chemotherapy and/or radiotherapy; preferably, a chemotherapeutic agent used in the chemotherapy received by the subject with the osteosarcoma and/or chondrosarcoma includes methotrexate, ifosfamide, cisplatin, and doxorubicin; and more preferably, a disease progresses after the subject with the osteosarcoma and/or chondrosarcoma has been treated with chemotherapy and/or radiotherapy.

12. Anlotinib or a pharmaceutically acceptable salt thereof for use according to claim 1 or 2, wherein a daily dose of said anlotinib or the pharmaceutically acceptable salt thereof administered to a patient is selected from 2 to 20 mg, 5 to 20 mg, 10 to 16 mg, or 10 to 14 mg; preferably, 8 mg, 10 mg, 12 mg, 14 mg, or 16 mg.

13. Anlotinib or a pharmaceutically acceptable salt thereof for use according to claim 1 or 2, wherein anlotinib or the pharmaceutically acceptable salt thereof is administered to a patient by means of intermittent administration, the intermittent administration includes an administration period and an off period, wherein a ratio of the administration period to the off period in terms of days is selected from the following ratios of 2 : 0.5 to 5, 2 : 0.5 to 3, 2 : 0.5 to 2, 2 : 0.5 to 1; preferably, the means of intermittent administration is a continuous administration for 2 weeks and a withdrawal for 1 week.

14. Anlotinib or a pharmaceutically acceptable salt thereof for use according to claim 1, wherein anlotinib or the pharmaceutically acceptable salt thereof is administered to a patient with osteosarcoma or chondrosarcoma together with other antitumor drug(s) simultaneously or sequentially, said other antitumor drug includes an alkylating agent, a platinum complex, a fluoropyrimidine derivative, camptothecin and a camptothecin analog, an anthraquinon-based antitumor antibiotic, and taxanes; preferably, said other antitumor drug is cisplatin.

15. Anlotinib or a pharmaceutically acceptable salt thereof for use according to claim 1, wherein the drug comprises a therapeutically effective amount of anlotinib and a pharmaceutically acceptable carrier.

## Patentansprüche

1. Anlotinib oder ein pharmazeutisch annehmbares Salz davon, zur Verwendung bei der Hemmung eines Osteosarkoms und/oder eines Chondrosarkoms bei einem behandlungsbedürftigen Patienten.

2. Anlotinib oder ein pharmazeutisch annehmbares Salz davon, zur Verwendung als Potenziator von Cisplatin bei der Hemmung von Osteosarkom und/oder Chondrosarkom bei einem behandlungsbedürftigen Patienten.

3. Anlotinib oder ein pharmazeutisch annehmbares Salz davon zur Verwendung nach Anspruch 1 oder 2, wobei das pharmazeutisch annehmbare Salz von Anlotinib Anlotinib-Hydrochlorid, vorzugsweise Dihydrochlorid, ist.

4. Anlotinib oder ein pharmazeutisch annehmbares Salz davon zur Verwendung nach Anspruch 1 oder 2, wobei das Arzneimittel das Wachstum und/oder die Metastasierung und/oder Rezidive von Osteosarkomen und/oder Chondrosarkomen hemmt.

5. Anlotinib oder ein pharmazeutisch annehmbares Salz davon zur Verwendung nach Anspruch 4, wobei die Metastasierung des Osteosarkoms und/oder Chondrosarkoms Migration und/oder Invasion ist.

6. Anlotinib oder ein pharmazeutisch annehmbares Salz davon zur Verwendung nach Anspruch 1 oder 2, wobei das Osteosarkom ein primäres Osteosarkom und/oder ein sekundäres Osteosarkom ist.

7. Anlotinib oder ein pharmazeutisch annehmbares Salz davon zur Verwendung nach Anspruch 1 oder 2, wobei das Osteosarkom ein osteogenes Osteosarkom und/oder ein osteolytisches Osteosarkom ist.

8. Anlotinib oder ein pharmazeutisch annehmbares Salz davon zur Verwendung nach Anspruch 1 oder 2, wobei das Osteosarkom ein osteoblastisches Osteosarkom, ein chondroblastisches Osteosarkom und/oder ein fibroblastisches Osteosarkom ist.

9. Anlotinib oder ein pharmazeutisch annehmbares Salz davon zur Verwendung nach Anspruch 1 oder 2, wobei das Chondrosarkom ein entdifferenziertes Chondrosarkom und/oder ein gut differenziertes Chondrosarkom ist.

10. Anlotinib oder ein pharmazeutisch annehmbares Salz davon zur Verwendung nach Anspruch 1 oder 2, wobei das Osteosarkom und/oder Chondrosarkom ein fortgeschrittenes und/oder metastasierendes Osteosarkom und/oder Chondrosarkom ist; vorzugsweise ist das metastasierende Osteosarkom und/oder Chondrosarkom ein Osteosarkom und/oder Chondrosarkom mit Lungenmetastasen.

11. Anlotinib oder ein pharmazeutisch annehmbares Salz davon zur Verwendung nach Anspruch 1 oder 2, wobei ein Subjekt mit dem Osteosarkom und/oder Chondrosarkom mit Chemotherapie und/oder Strahlentherapie behandelt wurde; vorzugsweise umfasst ein Chemotherapeutikum, das in der Chemotherapie verwendet wurde, die das Subjekt mit dem Osteosarkom und/oder Chondrosarkom erhalten hat, Methotrexat, Ifosfamid, Cisplatin und Doxorubicin; und noch bevorzugter schreitet eine Krankheit fort, nachdem das Subjekt mit dem Osteosarkom und/oder Chondrosarkom mit Chemotherapie und/oder Strahlentherapie behandelt worden ist.

12. Anlotinib oder ein pharmazeutisch annehmbares Salz davon zur Verwendung nach Anspruch 1 oder 2, wobei eine tägliche Dosis des Anlotinibs oder des pharmazeutisch annehmbaren Salzes davon, die einem Patienten verabreicht wird, ausgewählt ist aus 2 bis 20 mg, 5 bis 20 mg, 10 bis 16 mg oder 10 bis 14 mg; vorzugsweise 8 mg, 10 mg, 12 mg, 14 mg oder 16 mg.

13. Anlotinib oder ein pharmazeutisch annehmbares Salz davon zur Verwendung nach Anspruch 1 oder 2, wobei Anlotinib oder das pharmazeutisch annehmbare Salz davon einem Patienten mittels intermittierender Verabreichung verabreicht wird, wobei die intermittierende Verabreichung einen Verabreichungszeitraum und einen Absetzungszeitraum einschließt, wobei ein Verhältnis des Verabreichungszeitraums zum Absetzungszeitraum in Form von Tagen aus den folgenden Verhältnissen von 2 : 0. 5 bis 5, 2 : 0,5 bis 3, 2 : 0,5 bis 2, 2 : 0,5 bis 1 ausgewählt ist; vorzugsweise ist das Mittel der intermittierenden Verabreichung eine kontinuierliche Verabreichung für zwei Wochen und ein Absetzen für eine Woche.

14. Anlotinib oder ein pharmazeutisch annehmbares Salz davon zur Verwendung nach Anspruch 1, wobei Anlotinib oder das pharmazeutisch annehmbare Salz davon einem Patienten mit Osteosarkom oder Chondrosarkom zusammen mit einem oder mehreren anderen Antitumorarzneimitteln gleichzeitig oder nacheinander verabreicht wird, wobei das andere Antitumorarzneimittel ein Alkylierungsmittel, einen Platinkomplex, ein Fluorpyrimidinderivat, Camptothecin und ein Camptothecinanalogon, ein Antitumor-Antibiotikum auf Anthrachinonbasis und Taxane umfasst; vorzugsweise ist das andere Antitumormittel Cisplatin.

15. Anlotinib oder ein pharmazeutisch annehmbares Salz davon zur Verwendung nach Anspruch 1, wobei das Arzneimittel eine therapeutisch wirksame Menge von Anlotinib und einen pharmazeutisch annehmbaren Träger umfasst.

## Revendications

1. Anlotinib ou son sel pharmaceutiquement acceptable, pour l'utilisation dans l'inhibition de l'ostéosarcome et/ou du chondrosarcome chez un patient ayant besoin de traitement.

2. Anlotinib ou son sel pharmaceutiquement acceptable, pour l'utilisation comme potentialisateur de la cisplatine dans l'inhibition de l'ostéosarcome et/ou du chondrosarcome chez un patient ayant besoin de traitement.

3. Anlotinib ou son sel pharmaceutiquement acceptable pour l'utilisation selon la revendication 1 ou 2, le sel pharmaceutiquement acceptable d'anlotinib étant le chlorhydrate d'anlotinib, préférablement le dichlorhydrate.

4. Anlotinib ou son sel pharmaceutiquement acceptable pour l'utilisation selon la revendication 1 ou 2, le médicament inhibant la croissance et/ou la métastase et/ou la récurrence d'un ostéosarcome et/ou d'un chondrosarcome.

5. Anlotinib ou son sel pharmaceutiquement acceptable pour l'utilisation selon la revendication 4, la métastase de l'ostéosarcome et/ou du chondrosarcome étant une migration et/ou une invasion.

6. Anlotinib ou son sel pharmaceutiquement acceptable pour l'utilisation selon la revendication 1 ou 2, l'ostéosarcome étant un ostéosarcome primaire et/ou un ostéosarcome secondaire.

7. Anlotinib ou son sel pharmaceutiquement acceptable pour l'utilisation selon la revendication 1 ou 2, l'ostéosarcome étant un ostéosarcome ostéogène et/ou un ostéosarcome ostéolytique.

8. Anlotinib ou son sel pharmaceutiquement acceptable pour l'utilisation selon la revendication 1 ou 2, l'ostéosarcome étant un ostéosarcome ostéoblastique, un ostéosarcome chondroblastique et/ou un ostéosarcome fibroblastique.

9. Anlotinib ou son sel pharmaceutiquement acceptable pour l'utilisation selon la revendication 1 ou 2, le chondrosarcome étant un chondrosarcome dédifférencié et/ou un chondrosarcome bien différencié.

10. Anlotinib ou son sel pharmaceutiquement acceptable pour l'utilisation selon la revendication 1 ou 2, l'ostéosarcome et/ou le chondrosarcome étant un ostéosarcome et/ou un chondrosarcome avancé et/ou métastasique ; préférablement, l'ostéosarcome et/ou le chondrosarcome métastasique étant un ostéosarcome et/ou un chondrosarcome avec des métastases pulmonaires.

11. Anlotinib ou son sel pharmaceutiquement acceptable pour l'utilisation selon la revendication 1 ou 2, un sujet souffrant d'ostéosarcome et/ou le chondrosarcome ayant été traité par chimiothérapie et/ou radiothérapie ; préférablement, un agent chimiothérapeutique utilisé dans la chimiothérapie reçue par le sujet souffrant d'ostéosarcome et/ou le chondrosarcome comprenant du méthotrexate, de l'ifosfamide, de la cisplatine, et de la doxorubicine ; et plus préférablement, une maladie progressant après que le sujet souffrant d'ostéosarcome et/ou du chondrosarcome a été traité par chimiothérapie et/ou radiothérapie.

12. Anlotinib ou son sel pharmaceutiquement acceptable pour l'utilisation selon la revendication 1 ou 2, une dose quotidienne dudit anlotinib ou son sel pharmaceutiquement acceptable administré à un patient étant sélectionnée parmi 2 à 20 mg, 5 à 20 mg, 10 à 16 mg, ou 10 à 14 mg ; préférablement, 8 mg, 10 mg, 12 mg, 14 mg, ou 16 mg.

13. Anlotinib ou son sel pharmaceutiquement acceptable pour l'utilisation selon la revendication 1 ou 2, l'anlotinib ou son sel pharmaceutiquement acceptable étant administré à un patient par administration intermittente, l'administration intermittente comprenant une période d'administration et une période hors administration, un rapport de la période d'administration à la période hors administration en termes de jours étant sélectionné parmi les rapports suivants de 2:0,5 à 5, de 2:0,5 à 3, de 2:0,5 à 2, de 2:0,5 à 1 ; préférablement, le moyen d'administration intermittente étant une administration continue de 2 semaines et une interruption d'1 semaine.

14. Anlotinib ou son sel pharmaceutiquement acceptable pour l'utilisation selon la revendication 1, l'anlotinib ou son sel pharmaceutiquement acceptable étant administré à un patient souffrant d'ostéosarcome ou de chondrosarcome conjointement à un autre(d'autres) médicament(s) antitumoral(aux) simultanément ou séquentiellement, ledit autre médicament antitumoral comprenant un agent d'alkylation, un complexe de platine, un dérivé fluoropyrimidine, de la camptothécine et un analogue de la camptothécine, un antibiotique antitumoral à base d'anthraquinone, et des taxanes ; préférablement, ledit autre médicament antitumoral étant la cisplatine.

15. Anlotinib ou son sel pharmaceutiquement acceptable pour l'utilisation selon la revendication 1, le médicament comprenant une quantité thérapeutiquement efficace d'anlotinib et un support pharmaceutiquement acceptable.
